# EUROPÄISCHE PATENTSCHRIFT

(11) **EP 3 614 923 B1**
(45) Veröffentlichungstag und Bekanntmachung des Hinweises auf die Patenterteilung: **30.03.2022**
(21) Anmeldenummer: 18721020.8
(22) Anmeldetag: 25.04.2018
(51) Int. Cl.: A61B 5/15, B01L 3/00, B01D 21/26, G01N 33/49

(54) **TRENNKÖRPER**
SEPARATION BODY
CORPS DE SÉPARATION

(30) Priorität: 26.04.2017 DE 102017108937
(43) Veröffentlichungstag der Anmeldung: 04.03.2020
(73) Patentinhaber: Sarstedt Aktiengesellschaft & Co. KG, 51588 Nümbrecht (DE)
(72) Erfinder: WEINSTOCK, Mark, 57612 Helmenzen (DE); WEGENER, Christian, 51588 Nümbrecht (DE); KARRENBERG, Ulrich, 51709 Marienheide (DE)
(74) Vertreter: Grosse, Wolf-Dietrich Rüdiger
(86) Internationale Anmeldenummer: PCT/EP2018/060602
(87) Internationale Veröffentlichungsnummer: WO 2018/197565

(56) Entgegenhaltungen:
- EP-A1- 0 753 741
- EP-A2- 0 098 150
- WO-A1-2010/132783
- JP-A- H09 222 427
- US-A- 5 632 905
- US-B1- 6 280 400

## Beschreibung

Die Erfindung betrifft einen Trennkörper zum Trennen einer ersten von einer zweiten Phase einer Flüssigkeit in einem rohrförmigen Behälter. Gemeint sind insbesondere Trennkörper zum Trennen von Blutserum als erster Phase von Blutkuchen als zweiter Phase bei Blut als Flüssigkeit innerhalb eines Blutentnahmeröhrchens.

Im Stand der Technik sind Blutentnahmeröhrchen mit Trennkörpern grundsätzlich bekannt. Im Auslieferungszustand der Blutentnahmeröhrchen sind die Trennkörper in einer Ausgangsposition fixiert. Wenn Blut über einen Zulauf in das Blutentnahmeröhrchen hineinfließt, wird der Trennkörper von dem Blut umströmt oder durchströmt; in der Ausgangsposition stellt der Trennkörper jedenfalls keine Dichtung für das Blut innerhalb des Blutentnahmeröhrchens dar. Zur medizinischen Analyse ist es erforderlich, dass das Blut in zwei Bestandteile, nämlich Blutserum und Blutkuchen aufgetrennt wird. Zu diesem Zweck wird das Blutentnahmeröhrchen mit dem darin befindlichen Blut zentrifugiert. Der schwerere Blutkuchen setzt sich dann aufgrund der Zentrifugation in dem bodennahen Volumenbereich des Blutentnahmeröhrchens ab, während das leichtere Blutserum auf dem Blutkuchen aufschwimmt. Unter Einwirkung der Zentrifugalkraft löst sich der Trennkörper aus seiner Ausgangsposition und wandert in eine Abdichtposition. Weil die Dichte des gesamten Trennkörpers in einem Wertebereich zwischen der Dichte des Blutserums und der Dichte des Blutkuchens liegt, positioniert sich der Trennkörper automatisch genau an der Phasengrenze zwischen Blutserum und Blutkuchen. Diese Position wird auch Abdichtposition genannt, weil der Trennkörper in dieser Position mit seinem Dichtrand umlaufend dichtend an der Innenseite des rohrförmigen Proberöhrchens anliegt und somit das Blutserum sauber von dem Blutkuchen trennt. Der Trennkörper hält diese Abdichtposition auch nach Ende des Zentrifugierens bei, so dass das Blutserum und der Blutkuchen für eine Laboruntersuchung getrennt zur Verfügung stehen.

Trennkörper sind offenbart, z. B. in der internationalen Patentanmeldung WO 2010/132783 A1. Die dort beschriebenen Trennkörper weisen jeweils einen aus elastischem Material gefertigten Schwimmkörper mit einem in der Draufsicht kreisförmigen umlaufenden Dichtrand auf, wobei der Dichtrand zum dichtenden Anliegen an der Innenseite eines rohrförmigen Probenbehälters in einer Abdichtposition ausgebildet ist. An der Unterseite des Schwimmkörpers ist jeweils ein Ballastkörper befestigt. Die Dichte des Ballastkörpers ist jeweils größer als die Dichte des Schwimmkörpers und die Dichte des gesamten Trennkörpers liegt in einem Wertebereich zwischen der Dichte der ersten Phase und der Dichte der zweiten Phase der Flüssigkeit.

Aus dem Stand der Technik in Form der Druckschrift WO 2016/076911 A1 ist eine Trenneinheit für die Trennung einer Flüssigkeit in eine erste leichte und in eine zweite schwerere Phase unter Anwendung von Zentrifugalkraft bekannt, wobei die Flüssigkeit Blut sein kann. Ein rohrförmiger Behälter weist einen Trennkörper auf, wobei der Trennkörper im oberen Bereich einen Schwimmkörper und im unteren Bereich einen Ballastkörper aufweist. Der Trennkörper ist ausgebildet zum dichtenden Anliegen an der Innenseite des rohrförmigen Behälters. Die Dichte des Ballastkörpers ist dabei größer als die Dichte des Schwimmkörpers und die Dichte des Trennkörpers liegt zwischen der Dichte der ersten Phase und der Dichte der zweiten Phase der zu trennenden Flüssigkeit.

In der Druckschrift DE 699 31 584 T2 wird eine Vorrichtung zum Trennen einer Fluidprobe unter Zentrifugalkraft in eine Phase mit höherem spezifischen Gewicht und eine Phase mit niedrigerem spezifischen Gewicht beschrieben, wobei die Fluidprobe eine Blutprobe sein kann. Die Vorrichtung weist ein Separatorelement (Trennkörper) auf, das in einem zylindrischen Röhrchen angeordnet ist. Das Separatorelement weist einen Schwimmer im oberen Bereich und ein Ballastelement im unteren Bereich auf sowie einen Dichtungskörper zum dichtenden Anliegen an der Innenseite des Röhrchens. Die Dichte des Ballastelements ist dabei größer als die Dichte des Schwimmers und die Gesamtdichte des Separatorelements liegt zwischen der Dichte der erste Phase und der Dichte der zweiten Phase und der zu trennenden Flüssigkeit.

Die Druckschrift DE 600 23 823 T2 beinhaltet eine Vorrichtung zur Trennung einer flüssigen Probe (z. B. Blut) in eine erste Phase hoher Dichte und in eine Phase niedriger Dichte unter Einwirkung von Zentrifugalkraft. In einem Röhrchen mit zylindrischer Seitenwand ist ein Separator (Trennkörper) angeordnet, der im oberen Bereich einen Schwimmer und im unteren Bereich einen Ballastteil aufweist sowie ein Faltenbalg zum dichtenden Anliegen an der Innenseite des Röhrchens. Die Dichte des Ballastteils ist dabei größer als die Dichte des Schwimmers und die Gesamtdichte des Separators liegt zwischen den Dichten der ersten Phase und der zweiten Phase der zu trennenden Flüssigkeit.

Die Druckschrift US 5,632,905 A betrifft die Trennung einer Blutprobe in eine leichtere und eine schwerere Phase durch Zentrifugieren in einem Röhrchen. In dem Röhrchen ist ein Trennkörper angeordnet. Der Trennkörper hat eine scheibenförmige Form und liegt in einer Abdichtposition an der Phasengrenze zwischen leichterer und schwererer Phase.

Der Erfindung liegt die Aufgabe zugrunde, einen alternativen Trennkörper zum Trennen einer ersten von einer zweiten Phase einer Flüssigkeit in einem rohrförmigen Behälter bereitzustellen.

Diese Aufgabe wird im Hinblick auf den Trennkörper durch den Gegenstand des Anspruchs 1 gelöst. Demnach ist der Trennkörper dadurch gekennzeichnet, dass der scheibenförmige Schwimmkörper - an seinem Rand verteilt und abwechselnd zu den Fingern - Verdickungen als Auftriebskörper aufweist; und die Auftriebskörper auf der den Fingern gegenüberliegenden Seite des scheibenförmigen Schwimmkörpers angeordnet sind.

Im Auslieferungszustand bzw. in seiner Ausgangsposition ist der Trennkörper in dem rohrförmigen Behälter lösbar eingeklemmt. Er sitzt dann quer in dem Behälter. Dabei drückt der Ballastkörper mit seinem dem Schwimmkörper abgewandten Ende der Finger gegen die Innenseite des rohrförmigen Behälters. Anders ausgedrückt, stützt sich der Trennkörper in dieser Ausgangsposition wie beschrieben gegen den rohrförmigen Behälter ab.

Die beanspruchte lichte Ausbildung des Trennkörpers und insbesondere seines Ballastkörpers in Form von Fingern gewährleistet vorteilhafterweise, dass der Trennkörper in seiner Ausgangsposition im Auslieferungszustand den rohrförmigen Behälter nicht für die Flüssigkeit abdichtet, sondern dass der Trennkörper von der in den Behälter einströmenden Flüssigkeit umströmt werden kann, so dass die Flüssigkeit in unterhalb des Trennkörpers befindliche Volumenbereiche des rohrförmigen Behälters einströmen kann.

Unter Einwirkung einer Kraft, insbesondere der Zentrifugalkraft löst sich der Trennkörper aus seiner Ausgangsposition und wandert dann in eine Abdichtposition innerhalb des rohrförmigen Behälters. Die beanspruchte, vorzugsweise gleichmäßige Verteilung der Finger des Ballastkörpers am Umfang des Schwimmkörpers bewirkt vorteilhafterweise, dass sich der Trennkörper unter Einwirkung der Kraft - über seinen Umfang verteilt - gleichmäßig verschlankt, d. h. sein Durchmesser reduziert sich gleichmäßig über den Umfang. Dadurch wird die besagte Wanderung des Trennkörper insofern begünstigt, als dass er bei seiner Wanderung nicht an der Innenseite des Behälters festklemmen kann.

Allgemein gilt: Die Dichte der zweiten Phase der Flüssigkeit ist größer als die Dichte der ersten Phase der Flüssigkeit. Für Blut als Flüssigkeit bedeutet dies, dass der Blutkuchen als zweite Phase eine größere Dichte aufweist als das Blutserum, welches der ersten Phase entspricht. Nach einer Zentrifugation schwimmt deshalb das Blutserum auf dem Blutkuchen auf. Die Dichte des gesamten Trennkörpers liegt in einem Wertebereich zwischen der Dichte der ersten Phase und der Dichte der zweiten Phase der Flüssigkeit. Deshalb positioniert sich der Trennkörper in der Abdichtposition immer auf der Phasengrenze zwischen den beiden Phasen.

Sofern nichts anderes gesagt ist, wird der Trennkörper nachfolgend in einer Normalposition beschrieben. In dieser Normalposition ist der Ballastkörper unterhalb des Schwimmkörpers angeordnet. Der Schwerpunkt des Schwimmkörpers, der Schwerpunkt des Ballastkörpers und der Schwerpunkt des gesamten Trennkörpers liegen alle auf einer vertikalen Linie. Die nachfolgend verwendeten Begriffe wie vertikal, horizontal, unterhalb, Seitenansicht und Draufsicht etc. beziehen sich alle auf diese Normalposition. Die Abdichtposition entspricht der Normalposition bei senkrecht stehendem rohrförmigem Behälter.

Der beanspruchte scheibenförmige Schwimmkörper weist an seinem Rand verteilte Auftriebskörper auf. Diese Auftriebskörper sind in Umfangsrichtung abwechselnd zu den Fingern angeordnet. Die Auftriebskörper sind auf der Oberseite des scheibenförmigen Schwimmköpers aufragend angeordnet, während die Finger, wie gesagt von der Unterseite des scheibenförmigen Schwimmkörpers nach unten abstehen. Weil sich die Auftriebskörper und die Finger in jeweils entgegengesetzte Richtungen erstrecken, hat dies den Vorteil, dass die Schwerpunkte des Schwimmkörpers und des Ballastkörpers einen großen Abstand zueinander aufweisen. Dies ist vorteilhaft für eine stabile Lage des Trennkörpers an der Phasengrenze und damit für eine sichere Trennung der beiden Phasen der Flüssigkeit innerhalb des rohrförmigen Behälters.

Gemäß einem ersten Ausführungsbeispiel weist der scheibenförmige Schwimmkörper in seiner Mitte eine Verdickung auf; dieser dient als Auftriebskörper und gewährleistet, dass der Trennkörper innerhalb des rohrförmigen Behälters auf der schwereren zweiten Phase der Flüssigkeit aufschwimmt

Wenn der scheibenförmige Schwimmkörper nicht sphärisch verformt ist, verläuft sein Seitenrand - in einer Seitenansicht gesehen - gerade, vorzugsweise horizontal. Alternativ kann der scheibenförmige Schwimmkörper auch sphärisch verformt sein; in der Seitenansicht verläuft sein Dichtrand dann wellenförmig mit Wellenbergen und Wellentälern. Vorzugsweise sind dann die Finger des Ballastkörpers jeweils im Bereich der Wellentäler und die Verdickungen bzw. Auftriebskörper jeweils im Bereich der Wellenberge an dem Rand des Schwimmkörpers angeordnet. Diese besondere Ausgestaltung des Trennkörpers begünstigt vorteilhafterweise eine Verschlankung des Trennkörpers wenn sich dieser - unter Einwirkung der Zentrifugalkraft - aus seiner Ausgangsposition in die Abdichtposition bewegt.

Schließlich können zumindest einzelne der Finger an ihren freien Enden ein Haftelement aufweisen, welches sich durch eine vorbestimmte Haftreibungszahl an seiner Oberfläche auszeichnet.

Das Haftelement kann aus demselben Material wie der Schwimmkörper und vorzugsweise einstückig mit dem Schwimmkörper ausgebildet sein. Das Haftelement ist dann vorteilhafterweise fertigungstechnisch kostengünstig und einfach zu realisieren. Die einstückige Ausbildung mit dem Schwimmkörper kann beispielsweise einfach dadurch realisiert werden, dass in einer Spritzgussform für den Trennkörper an der Außenseite oder im Innern des Ballastkörpers kleine Kanäle vorgesehen werden, welche den Schwimmkörper mit dem Haftelement verbinden. Beim Spritzgießen des Schwimmkörpers wird dann das Material des Schwimmkörpers durch die Kanäle auch in die Hohlräume für die Haftelemente hineingespritzt. Nach Entfernen der Spritzgussform verbleibt das Material des Schwimmkörpers im Bereich der Kanäle der Spritzgussform an der Außenseite oder im Innern des Ballastkörpers in Form von Stegen erhalten, welche den Schwimmkörper mit dem Haftelement einstückig verbinden. Die Stege sind lediglich optional. Der Schwimmkörper und die Haftelemente können auch unabhängig voneinander als Einzelelemente ausgebildet sein, was aber fertigungstechnisch aufwendiger ist.

Weitere vorteilhafte Ausgestaltungen des Trennkörpers sind Gegenstand der abhängigen Ansprüche.

Der Beschreibung sind vier Figuren beigefügt, wobei
- Figuren 1a und 1b: einen Trennkörper gemäß der Erfindung mit scheibenförmigem Schwimmkörper
- Figur 2: den Trennkörper nach Figur 1 in einem rohrförmigen Behälter zeigt.

Die Erfindung wird nachfolgend unter Bezugnahme auf die genannten Figuren in Form von Ausführungsbeispielen detailliert beschreiben. In allen Figuren sind gleiche technische Elemente mit gleichen Bezugszeichen bezeichnet.

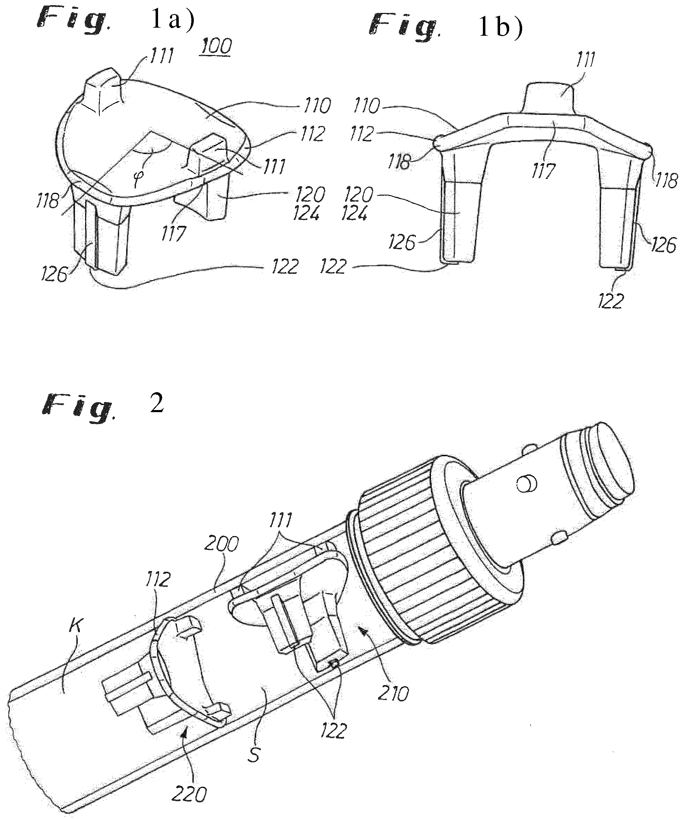

Figur 1 zeigt den erfindungsgemäßen Trennkörper 100 in einer erfindungsgemäßen Ausgestaltung. Figur 1a zeigt ihn in perspektivischer Ansicht, und Figur 1b zeigt ihn in einer Seitenansicht. Auch hier ist der Schwimmkörper 110 grundsätzlich scheibenförmig ausgebildet, allerdings ist er sphärisch verformt. Deshalb ist der umlaufende Rand 112 von der Seite betrachtet wellenförmig mit Wellentälern 118 und Wellenbergen 117 ausgebildet. Von der Unterseite des scheibenförmigen Schwimmkörpers 110 stehen jeweils im Bereich der Wellentäler 118 Ballastkörper 120 in Form von Fingern 124 nach unten ab. Vorzugsweise jeder dieser Finger weist an seinem dem Schwimmkörper 110 abgewandten Ende das besagte Haftelement 122 auf. Im Bereich der Wellenberge 117 sind auf der Oberseite des scheibenförmigen Schwimmkörpers Materialansammlungen ausgebildet; diese wirken als zusätzliche Auftriebskörper 111. Die Finger 124 und die Auftriebskörper 113 sind bei dem in Figur 1 gezeigten Ausführungsbeispiel jeweils mit einem Umfangswinkelabstand von ϕ=90° an der Peripherie des scheibenförmigen Schwimmkörpers abwechselnd verteilt angeordnet.

Diese spezielle Anordnung von Auftriebskörpern und Ballastkörpern ist besonders vorteilhaft für die Wanderung des Trennkörpers von der Ausgangsposition in die Abdichtposition. Die dabei auf den Trennkörper einwirkende Kraft, insbesondere die Zentrifugalkraft bewirkt, dass sich der Schwimmkörper 110 gegenüber seiner Ausgangsform noch stärker sphärisch verformt. Dies gilt deshalb, weil dann der Auftrieb der Auftriebskörper 111 die Wellenberge 117 noch weiter nach oben zieht und weil gleichzeitig die Finger 124 des Ballastkörpers im Bereich der Wellentäler 118 weiter nach unten gezogen werden. Aufgrund dieser noch stärkeren sphärischen Verformung verschlankt der Trennkörper 100 und der Dichtrand 112 des Schwimmkörpers liegt dann nicht mehr dichtend an der Innenseite des rohrförmigen Behälters an. Der Schwimmkörper kann deshalb, wie gewünscht, auf seinem Weg in die Abdichtposition 220 von der Flüssigkeit bzw. dem Blut umströmt werden.

Figur 2 zeigt den Trennkörper gemäß Figur 1 im Inneren des rohrförmigen Behälters 200. Im Auslieferungszustand befindet sich der Trennkörper in seiner Ausgangsposition 210. Er stützt sich dann einerseits mit den freien Enden seiner Finger, gegebenenfalls mit den daran befindlichen Haftelementen 122, und andererseits mit den freien Enden der Auftriebskörper 111 an der Innenseite des rohrförmigen Behälters 200 ab. Die freien Enden der Auftriebskörper 111 sind deshalb vorzugsweise entsprechend dem Innenradius des rohrförmigen Behälters 200 abgerundet. In der Ausgangsposition 210 kann der Trennkörper 100, wie oben beschrieben, insbesondere aufgrund seiner lichten Ausgestaltung von in den rohrförmigen Behälter einfließendem Blut umströmt werden, sodass das Blut auch in tiefere Volumenbereiche des rohrförmigen Behälters gelangen kann.

Erst unter Einwirkung der Zentrifugalkraft löst sich der Trennkörper 100 aus seiner Ausgangsposition 210 und wandert in die Abdichtposition 220. Dabei dreht er sich um 90°. Erst wenn der Trennkörper 100 nicht mehr unter der Einwirkung der Zentrifugalkraft steht, verformt er sich wieder zurück in seinen Ausgangszustand. Sein Dichtrand 112 liegt dann in der Abdichtposition 220 in Umfangsrichtung R überall dichtend an der Innenseite des rohrförmigen Behälters 200 an und separiert auf diese Weise die beiden Phasen der Flüssigkeit bzw. des Blutes wirksam voneinander.

## Patentansprüche

1. Trennkörper (100) zum Trennen einer ersten von einer zweiten Phase einer Flüssigkeit, insbesondere Blutserum (S) von Blutkuchen (K) bei Blut als Flüssigkeit, unter Zentrifugalkraft in einem rohrförmigen Behälter (200); aufweisend:
einen aus elastischem Material gefertigten Schwimmkörper (110) mit einem in der Draufsicht umlaufenden Dichtrand (112) zum dichtenden Anliegen an der Innenseite des rohrförmigen Behälters (200) in einer Abdichtposition (220); und
mindestens einen an der Unterseite des Schwimmkörpers (110) befestigten Ballastkörper (120);
wobei die Dichte des Ballastkörpers (120) größer ist als die Dichte des Schwimmkörpers;
wobei die Dichte des gesamten Trennkörpers (100) in einem Wertebereich zwischen der Dichte der ersten Phase und der Dichte der zweiten Phase der Flüssigkeit liegt;
wobei der Schwimmkörper (110) scheibenförmig ausgebildet ist; und
wobei der Ballastkörper (120) in Form einer Mehrzahl von Fingern (124) ausgebildet ist, die sich von der Unterseite des scheibenförmigen Schwimmkörpers (110) - an dessen Rand verteilt - weg erstrecken **dadurch gekennzeichnet, dass**
der scheibenförmige Schwimmkörper (110) - an seinem Rand verteilt und abwechselnd zu den Fingern - Verdickungen als Auftriebskörper (111) aufweist; und
die Auftriebskörper (111) auf der den Fingern gegenüberliegenden Seite des scheibenförmigen Schwimmkörpers (110) angeordnet sind.

2. Trennkörper (110) nach Anspruch 1,
**dadurch gekennzeichnet, dass**
der scheibenförmige Schwimmkörper (110) in seiner Mitte eine Verdickung als Auftriebskörper (111) aufweist.

3. Trennkörper (100) nach Anspruch 1,
**dadurch gekennzeichnet, dass**
die Finger (124) und die Auftriebskörper (111) in einem vorzugsweise gleichen Umfang-Winkelabstand (ϕ) abwechselnd an dem Rand des Schwimmkörpers (110) verteilt angeordnet sind.

4. Trennkörper (100) nach Anspruch 3,
**dadurch gekennzeichnet, dass**
an dem Rand des Schwimmkörpers zwei Auftriebskörper (111) und zwei Finger (124) angeordnet sind, wobei zwischen einem der Auftriebskörper und einem der Finger (124) jeweils ein Winkelabstand (ϕ) von 90° besteht.

5. Trennkörper (100) nach einem der vorangegangenen Ansprüche,
**dadurch gekennzeichnet, dass**
der Dichtrand (112) gerade, vorzugsweise horizontal verlaufend ausgebildet ist;

6. Trennkörper (100) nach einem der Ansprüche 1 bis 4,
**dadurch gekennzeichnet, dass**
der scheibenförmige Schwimmkörper (110) sphärisch verformt mit einem - in der Seitenansicht - wellenförmigen Dichtrand (112) mit Wellenbergen (117) und Wellentälern (118) ausgebildet ist; und
die Finger (124) des Ballastkörpers (120) jeweils im Bereich der Wellentäler (118) und die Verdickungen (111) des Schwimmkörpers (110) jeweils im Bereich der Wellenberge (117) an dem Rand des Schwimmkörpers (110) angeordnet sind.

7. Trennkörper (100) nach einem der vorangegangenen Ansprüche,
**dadurch gekennzeichnet, dass**
mindestens einer der Finger (124) an seinen freien, dem Schwimmkörper (110) abgewandten Ende ein Haftelement (122) aufweist mit einer vorbestimmten Haftreibungszahl an seiner Oberfläche aufweist.

8. Trennkörper (100) nach Anspruch 7,
**dadurch gekennzeichnet, dass**
das Haftelement (122) aus demselben Material wie der Schwimmkörper, vorzugsweise einstückig mit diesem ausgebildet ist.

## Claims

1. A separator (100) for separating a first phase from a second phase of a fluid, more particularly serum (S) from blood clots (K) in the case of blood as the fluid, under centrifugal force in a tube-shaped container (200), comprising:
a floating element (110) made of an elastic material with, when viewed from above, a circumferential sealing edge (112) for sealing contact on the inner side of the tube-like container (200) in a sealing position (220); and
fastened on the underside of the floating element (110), at least one ballast element (120);
wherein the density of the ballast element (120) is greater than the density of the floating element;
wherein the density of the entire separator (100) lies within a value range between the density of the first phase and the density of the second phase of the fluid;
wherein the floating element (110) is designed in a disk shape; and wherein the ballast element (120) is designed in the form of a plurality of fingers (124) which extend away from the underside of the disk-shaped floating element (110) - distributed on its edge,
**characterised in that**
distributed on its edge and alternating with the fingers, the disk-shaped floating element (110) comprises thickened portions as buoyancy elements (111); and
the buoyancy elements (111) are arranged on the side of the disk-shaped floating element (110) opposite the fingers.

2. The separator (100) according to claim 1, **characterised in that**
the disk-shaped floating element (110) has a thickened portion as a buoyancy element (111) at is centre.

3. The separator (100) according to claim 1, **characterised in that** the
fingers (124) and the buoyancy elements (111) are arranged distributed alternately on the edge of the floating element (110) at a preferably identical circumferential angular distance (ϕ).

4. The separator (100) according to claim 3, **characterised in that**
arranged on the edge of the floating element there are two buoyancy elements (111) and two fingers (124), wherein between one of the buoyancy elements and one of the fingers (124) there is an angular distance (ϕ) of 90° in each case.

5. The separator (100) according to any one of the preceding claims,
**characterised in that**
the sealing edge (112) designed to be straight, preferably extending horizontally.

6. The separator (100) according to any one of claims 1 to 4,
**characterised in that** the disk-shaped floating element (110) is designed as spherically deformed with, seen from the side, a wave-shaped sealing edge (112) with wave peaks (117) and wave troughs (118); and
the fingers (124) of the ballast element (120) are each arranged in the area of the wave troughs (118) and the thickened portions (111) of the floating element (110) are each arranged in the area of the wave peaks (117) of the edge of the floating element (110).

7. The separator (100) according to any one of the preceding claims,
**characterised in that**
at its free end facing away from the floating element (110), at least one of the fingers (124) has an adhesion element (122) with a predetermined stiction index on its surface.

8. The separator (100) according to claim 7, **characterised in that**
the adhesion element (122) is made of the same material as the floating element, preferably in one piece therewith.

## Revendications

1. Séparateur (100) pour séparer une première phase d'une seconde phase d'un liquide, en particulier de sérum sanguin (S) de caillots sanguin (K) dans du sang en tant que liquide, par force centrifuge dans un récipient en forme de tube (200) ;
présentant :
un corps flottant (110) fabriqué en matériau élastique comprenant un bord étanche (112) périphérique en vue de dessus, destiné à reposer en étanchéité sur la face intérieure du récipient en forme de tube (200) dans une position d'étanchéité (220) ; et
au moins un corps de ballast (120) fixé sur la sous-face du corps flottant (110) ;
dans lequel la densité du corps de ballast (120) est supérieure à la densité du corps flottant ;
dans lequel la densité de l'ensemble du séparateur (100) repose dans une plage de valeurs entre la densité de la première phase et la densité de la seconde phase du liquide ;
dans lequel le corps flottant (110) est conçu en forme de disque ; et
dans lequel le corps de ballast (120) est conçu sous forme d'une pluralité de doigts (124) qui s'éloignent de la sous-face du corps flottant (110) en forme de disque, répartis sur son bord, **caractérisé en ce que**
le corps flottant (110) en forme de disque présente des épaississements en tant que corps de portance (111), répartis sur son pourtour et en alternance avec les doigts ;
les corps de portance (111) sont disposés sur le côté du corps flottant (110) en forme de disque opposé aux doigts.

2. Séparateur (100) selon la revendication 1, **caractérisé en ce que** le corps flottant (110) en forme de disque présente un épaississement en tant que corps de portance (111) en son centre.

3. Séparateur (100) selon la revendication 1, **caractérisé en ce que** les doigts (124) et les corps de portance (111) sont disposés répartis par alternance sur le bord du corps flottant (110) dans un écart angulaire périphérique (φ) de préférence égal.

4. Séparateur (100) selon la revendication 3, **caractérisé en ce que** deux corps de portance (111) et deux doigts (124) sont disposés sur le bord du corps flottant, dans lequel il y a un écart angulaire (φ) respectif de 90° entre un des corps flottants et un des doigts (124).

5. Séparateur (100) selon l'une des revendications précédentes, **caractérisé en ce que** le bord d'étanchéité (112) est conçu droit, de préférence horizontal.

6. Séparateur (100) selon l'une des revendications 1 à 4, **caractérisé en ce que** le corps flottant (110) en forme de disque est sphérique avec un bord d'étanchéité (112) ondulé (en vue latérale) avec des crêtes (117) et des creux (118) ; et
les doigts (124) du corps de ballast (120) sont disposés respectivement au niveau des creux (118) et les épaississements (111) du corps flottant (110) respectivement au niveau des crêtes (117) sur le bord du corps flottant (110).

7. Séparateur (100) selon l'une des revendications précédentes, **caractérisé en ce qu'**au moins un des doigts (124) présente un élément d'adhérence (122) sur son extrémité libre tournée vers le corps flottant (110) avec un nombre de frottement par adhérence prédéfini sur sa surface.

8. Séparateur (100) selon la revendication 7, **caractérisé en ce que** l'élément d'adhérence (122) est conçu dans le même matériau que le corps flottant, est conçu de préférence d'une seule pièce avec celui-ci.
